# EUROPÄISCHE PATENTANMELDUNG

(11) **EP 0 639 346 A1**
(43) Veröffentlichungstag der Anmeldung: **22.02.1995**
(21) Anmeldenummer: 94810441.9
(22) Anmeldetag: 25.07.1994
(51) Int. Cl.: A61B 3/15, A61B 3/135

(54) **Verfahren und Gerät zum Untersuchen des Auges**

(30) Priorität: 16.08.1993 CH 2436/93
(71) Anmelder: HAAG-STREIT AG Werkstätten für Präzisionsmechanik, CH-3098 Köniz (CH)
(72) Erfinder: Widmer, Hansruedi, CH-3145 Niederscherli (CH); Stucki, Jürg, CH-3515 Oberdiessbach (CH); Bernau, Werner, Dr., CH-3098 Köniz (CH)
(74) Vertreter: AMMANN PATENTANWAELTE AG BERN

(57) **Zusammenfassung**

Bei der Beobachtung oder Aufnahme des Endothels (E) der Hornhaut (9) mittels eines Lichtbüschels (Io) entsteht ein starker Frontreflex (I_{Front}) und ein sehr viel schwächerer Reflex (I_{Endothel}) am Endothel (E) der Hornhaut. Bei der Beobachtung und Aufnahme eines Bildes des Endothels stört der sehr nahe liegende Frontreflex erheblich. Um diesen Nachteil zu beheben, ist eine verstellbare Blende (7) vorgesehen, welche einseitig in den Strahlengang der Abbildungsoptik eingeführt werden kann, um den Frontreflex (I_{Front}) abzublenden.

## Beschreibung

Die vorliegende Erfindung betrifft ein Verfahren und ein Gerät zum Untersuchen des Auges, insbesondere des Hornhautendothels, mittels eines in das Auge geworfenen Lichtbüschels, vorzugsweise eine Spaltbeleuchtung, und Betrachten bzw. Aufnahme mittels einer Abbildungsoptik. Bei solchen Augenuntersuchungen mittels einer Beleuchtung, insbesondere Spaltbeleuchtung, entsteht an der Hornhaut ein starker Frontreflex, der bei der Beobachtung oder der Aufnahme des gewünschten Bereiches im Augeninneren stören kann. Das ist insbesondere der Fall bei der Untersuchung des Hornhautendothels, d. h. der Zellen an der Innenseite der Hornhaut, wobei die Zellendichte für den Ophthalmologen ein wichtiger Parameter für die Beurteilung des Zustandes der Cornea darstellt. Für diese Untersuchung stehen bisher Spezialgeräte zur Verfügung, und zwar solche, die in direktem Kontakt mit der Hornhautvorderfläche stehen und solche, welche das Endothel ohne Berührung des Auges aufnehmen bzw. abbilden. Bei der Beobachtung bzw. Abbildung des Hornhautendothels liegt der erwähnte Frontreflex von der Vorderseite der Hornhaut sehr nahe bei dem viel schwächeren Reflex vom Hornhautendothel. Messungen ergeben, dass der überwiegende Teil des einfallenden Lichtes an der Hornhautvorderfläche reflektiert werden, d. h. dass der erwähnte Frontreflex sehr viel lichtstärker ist als der Hintergrundreflex. Da bei der Hornhautendothel-Untersuchung diese beiden Reflexe sehr nahe beieinander liegen, wird bei der Beobachtung der extreme Helligkeitsunterschied zwischen dem Frontreflex und dem Hintergrundreflex durch das Gehirn unterdrückt. Bei der Aufnahme des Bildes des Beleuchtungsspaltes durch das Mikroskop mit einer Videokamera oder einer Kleinbildkamera überblendet der Frontreflex den Endothelreflex so stark, dass die Zellschichten des an sich schon kontrastarmen Endothelbildes nicht mehr zu erkennen sind.

Hier will die vorliegende Erfindung Abhilfe schaffen, und zwar dadurch, dass der Frontreflex von der Aussenseite der Hornhaut abgeblendet wird. Vorzugsweise kann dabei der Frontreflex als Such- und Einstellhilfe benützt und dann zur Beobachtung abgeblendet werden. Das erfindungsgemässe Gerät ist demnach mit einer quer zum Strahlengang der Abbildungsoptik einstellbaren Blende ausgerüstet. Diese einstellbare Blende gestattet es, den Frontreflex abzublenden oder nicht und insbesondere eine Feineinstellung derart vorzunehmen, das der Frontreflex gerade genügend abgeblendet ist, dass gewünschte Bild des Hornhautendothels dagegen gut sichtbar bleibt.

Die Erfindung wird nun anhand der Zeichnung näher erläutert:
- Fig. 1: zeigt eine Gesamtansicht des erfindungsgemässen Gerätes
- Fig. 2: zeigt in starker Vergrösserung den Beleuchtungsstrahl, den Frontreflex und den Endothelreflex,
- Fig. 3: zeigt die erfindungsgemässe Blende in unwirksamer Stellung,
- Fig. 3a: zeigt das dabei entstehende Bild,
- Fig. 4: zeigt die Blende in wirksamer Stellung und
- Fig. 4a: zeigt das dabei entstehende Bild.

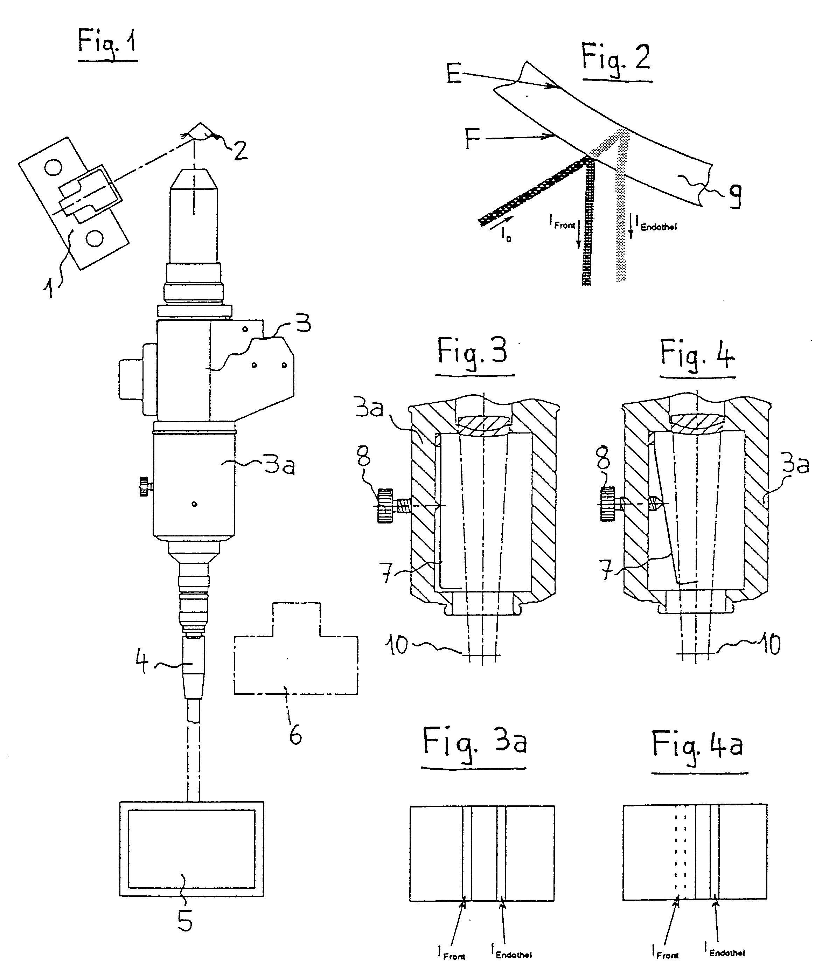

Figur 1 zeigt eine Spaltbeleuchtung 1, welche ein schmales Lichtbüschel auf das Auge 2 wirft. Das Auge wird mittels eines Mikroskops 3 beobachtet, wobei am Mikroskop eine Videokamera 4 montiert ist, welche mit einem Monitor 5 verbunden ist. Die Abbildung des Auges kann also laufend auf dem Monitor 5 beobachtet werden. Anstelle der Videokamera 4 könnte auch eine Kleinbildkamera 6, die in Figur 6 angedeutet ist, angeschlossen werden.

Im Video- bzw. Fototubus 3a ist eine als L-förmige Blattfeder ausgebildete Blende 7 eingebaut, welche mittels einer Stellschraube 8 aus einer unwirksamen Stellung gemäss Fig. 3 in eine wirksame Stellung im Strahlengang gemäss Fig. 4 verformt werden kann.

Figur 2 zeigt in starker Vergrösserung die Situation an der Hornhaut 9. Der eintretende Lichtstrahl Io wird grösstenteils an der Frontseite F der Hornhaut reflektiert und tritt als Reflex I_{Front} in das Mikroskop ein. Das durch die Hornhaut durchtretende Licht wird teilweise an der Rückseite bzw. dem Hornhautendothel E reflektiert und tritt als Endothelreflex I_{Endothel} in das Mikroskop ein. Wenn die beiden Reflexe gemäss Figur 3 unbehindert durchtreten können, entsteht in der Bildebene 10 das Bild gemäss Fig. 3a, d. h. beide Reflexe I_{Front} und I_{Endothel} erscheinen ungeschwächt auf dem Bild, was die eingangs erwähnten Nachteile mit sich bringt. Bei dieser Situation ist es allerdings leichter, das Mikroskop für die Betrachtung bzw. Aufnahme einzustellen, weil der starke Frontreflex eindeutig sichtbar ist. Zur eigentlichen Beobachtung bzw. Aufnahme des Endothels wird die Blende 7 gemäss Fig. 4 mittels der Schraube 8 möglichst nahe der Bildebene 10, in welcher entweder der Kamerachip oder ein fotographischer Film angeordnet ist, in den Strahlengang eingeführt, so dass derselbe einseitig abgeblendet wird und gemäss Fig. 4a ein Bild erscheint, in welchem der Frontreflex I_{Front} vollständig oder grösstenteils abgeblendet ist und die Beobachtung bzw. Aufnahme des Endothelreflexes I_{Endothel} nicht mehr behindert.

Die Blende 7 könnte auch anders ausgebildet sein und mittels eines Motors und einer Spindel fernsteuerbar sein.

## Patentansprüche

1. Verfahren zum Untersuchen des Auges, insbesondere des Hornhautendothels, mittels eines in das Auge geworfenen Lichtbüschels, vorzugsweise einer Spaltbeleuchtung und Betrachen bzw. Aufnahme mittels einer Abbildungsoptik, dadurch gekennzeichnet, dass der Frontreflex (I_{Front}) von der Aussenseite der Hornhaut (9) durch einseitiges Einschieben einer Blende (7) in den Strahlengang abgeblendet wird.

2. Verfahren nach Anspruch 1, dadurch gekennzeichnet, dass der Frontreflex (I_{Front}) als Such- und Einstellhilfe benützt und dann abgeblendet wird.

3. Gerät zur Durchführung des Verfahrens nach Anspruch 1, mit Mitteln (1, 3) zum Beleuchten und zum Abbilden des Auges bzw. von Teilen desselben, gekennzeichnet durch eine quer zum Strahlengang der Abbildungsoptik einstellbare Blende (7).

4. Gerät nach Anspruch 3, dadurch gekennzeichnet, dass die Blende (7) im Austrittsbereich der Abbildungsoptik, nahe der Bildebene (10) angebracht ist.

5. Gerät nach Anspruch 3 oder 4, dadurch gekennzeichnet, dass die Blende als Blattfeder ausgebildet ist, welche mittels einer Spindel oder Stellschraube (8) verformt werden kann.
